# EUROPEAN PATENT APPLICATION

(11) **EP 4 574 970 A1**
(43) Date of publication of application: **25.06.2025**
(21) Application number: 23307324.6
(22) Date of filing: 21.12.2023
(51) Int. Cl.: C12N 5/0789, A61K 35/12

(54) **POPULATION OF MYELOID-DERIVED SUPPRESSOR CELLS AND USES THEREOF**

(71) Applicant: Université de Lorraine, 54052 Nancy (FR); Centre National de la Recherche Scientifique, 75016 Paris (FR); Centre Hospitalier Régional Universitaire de Nancy, 54035 Nancy Cedex (FR)
(72) Inventor: D'AVENI, Maud, 54000 NANCY (FR); NOTARANTONIO, Anne-Béatrice, 57070 METZ (FR); RUBIO, Marie-Thérèse, 54000 NANCY (FR)
(74) Representative: Plasseraud IP

(57) **Abstract**

The present invention pertains to methods for producing myeloid-derived suppressor cells that are useful for the treatment of several diseases/conditions involving deleterious immune responses, such as graft versus host disease, graft rejection and autoimmune diseases. The method according to the present invention comprises culturing CD34⁺ cells with a specific combination of cytokines comprising TPO, G-CSF, GM-CSF, FLT-3L and SCF. The present invention further pertains to the myeloid-derived suppressor cells obtained by said method, which are qualified as CD34⁺, CD11 b⁺, CD33⁺ and CD127⁺ while being Lin⁻ cells.

## Description

### Technical Field

The present invention pertains to methods for producing myeloid-derived suppressor cells that are useful for the treatment of several diseases/conditions involving deleterious immune responses, such as graft versus host disease, graft rejection and autoimmune diseases.

### Background Art

Allogeneic hematopoietic stem cell transplantation (allo-HSCT) remains the only potentially curative treatment for certain blood cancers, particularly acute leukemias. Allo-HSCT is an immunotherapy that aims at eliminating recipient residual tumor cells by using the donor's immune system (GVL effect for Graft versus Leukemia). Unfortunately, this GVL effect is often associated with graft versus host disease (GVHD). GVHD occurs when the donor's T cells (from the graft) recognize the patient's healthy cells (the host) as foreign, and attack and damage them. GVHD can be fatal and is the first complication of allo-HSCT (it occurs in 30 to 50% of cases). The first-line treatment for GVHD is corticosteroid therapy. However, this therapy is complicated by numerous deleterious side effects such as diabetes, osteoporosis, hypertension, severe infections and risk of leukemia relapse. Furthermore, 30% of patients have a cortico-refractory GVHD and ruxolitinib is approved as a second line of treatment. Unfortunately, these patients have a very compromised life expectancy (<25% survival).

No immunosuppressive therapy known to date can reduce GVHD while preserving the GVL effect. Nevertheless, cell therapies are recognized as a promising approach for preventing/treating GVHD while maintaining the GVL effect, and the immunosuppressive properties of myeloid-derived suppressor cells (MDSCs) make them very interesting candidates.

MDSCs represent a heterogeneous population of cells of myeloid origin that expand during various pathological conditions, such as cancer and inflammation. They inhibit T cell proliferation following a no specific or allo-antigen specific stimulation.

To date, three teams have worked on the development of techniques to generate ex *vivo* myeloid suppressor cells in humans for curative purposes against GVHD:

Janikashvili et al., (Journal of Allergy and Clinical Immunology 135.6 (2015): 1614-1624 and oncoimmunology 2021 Feb 19;10(1):18880046) generated a subtype of monocytic myeloid suppressor cells from blood mononuclear cells of healthy donors cultured for 7 days in the presence of IL-6 and GM-CSF. They obtained mature CD33⁺CD11b⁺CD14⁺CD163⁺CD206⁺HLA-DR⁺ monocytes (named HuMoSC). In a mouse model, HuMoSC inhibit GVHD. However, they have not been tested for their immunogenicity (cells with strong HLA class II membrane expression) as we know that mature monocytes can release high amount of pro-inflammatory cytokines.

Casacuberta - Serra et al., (Immunology and cell biology 95.6 (2017): 538-548) proposed to obtain MDSCs from immature hematopoietic cells or mature monocytes. Hematopoietic progenitor cells (CD34⁺) cells were sorted from donor apheresis and frozen. Monocytes were obtained from donor peripheral blood, sorted and frozen. At the desired time, the frozen cells were thawed and cultured for 20 days with Stem Cell Factor (SCF) + thrombopoietin (TPO) + FLT3-L + GM-CSF + interleukin (IL)-6 to obtain both monocytic (CD33⁺ HLA-DR^{low} CD14⁺) and granulocytic (CD33⁺ HLA-DR^{low} CD15⁺) MDSCs. The cells obtained therefrom regulate allogeneic T cell proliferation *in vitro.* No *in vivo* regulatory properties have been demonstrated to date. The delay between thawing and obtention of the final therapeutic product is long with almost 3 weeks to wait.

Finally, Lim et al. (BBMT 2018 Dec;24(12):2381-2396) confirmed one year later (Park et al., Frontiers in immunology 10 (2019): 183) MDSC expansion from a cord blood unit (CBU). They investigated which cytokine combinations (GM-CSF / SCF), could induce human MDSCs from a CD34⁺ cell culture. They showed that they could obtain up to 10⁸ MDSCs (HLA-DR^{low} CD11b⁺ CD33⁺) from one unit of cord blood, and that the injection of these cells decreased the incidence of acute GVHD in a xenogeneic mouse model (NSG). Unfortunately, they had to wait 6 weeks of continuous culture to produce enough MDSCs, which seems tedious and too long to propose this production as a prophylaxis of severe acute GVHD.

There is thus an urgent need for efficient methods that allow a fast production of high-quality MDSCs that can be used for the treatment or prevention of conditions/diseases involving excessive immune responses.

### Summary

The invention is defined by the claims.

The present inventors have managed to develop a method that allows obtaining myeloid-derived suppressor cells having a strong *in vitro* and *in vivo* immunosuppressive property and that express CD34 as an immature phenotypic marker. The inventors have particularly identified a specific cytokine mixture that can be used for culturing CD34⁺ cells and rapidly obtain myeloid-derived suppressor cells.

The myeloid-derived suppressor cells obtained in the context of the present invention can efficiently reduce T lymphocyte response and present no/little immunogenicity. These properties make them an extremely powerful tool for the prevention/treatment of conditions involving deleterious T-lymphocyte responses. The cells obtained according to the method of the present invention can, for instance, efficiently prevent/treat GVHD. They can also be used for inducing immune-tolerance and replacing immunosuppressors used during and after organ transplantation or in autoinflammatory diseases.

The method according to the present invention is easy to implement and extremely quick: it allows obtaining myeloid-derived suppressor cells within only 7 days. It comprises culturing CD34⁺ cells with a specific cytokine mixture comprising thrombopoietin (TPO), granulocyte-colony stimulating factor (G-CSF), granulocyte-macrophage colony-stimulating factor (GM-CSF), FMS-like tyrosine kinase 3 ligand (FLT-3L) and stem cell Factor (SCF).

Therefore, according to a first aspect, the present invention pertains to a method for obtaining myeloid-derived suppressor cells, and to the myeloid-derived suppressor cells obtained therefrom, said method comprising a step of culturing CD34⁺ cells that have been isolated from a bone marrow graft or from peripheral blood or cord blood stem cells in a culture medium comprising cytokines, wherein said cytokines are TPO, G-CSF, GM-CSF, FLT-3L and SCF.

After characterization, the present inventors have demonstrated that the myeloid-derived suppressor cells obtained according to the method of the present invention can be qualified as CD34⁺, CD11b+, CD33+, CD127⁺ and Lin cells.

As mentioned above, these cells represent an extremely efficient tool for the treatment of conditions involving abnormal T-cell responses. Thus, the present invention also pertains to the myeloid-derived suppressor cells as described above for use as a medicament, particularly for the treatment of graft versus host disease, graft rejection, autoimmune and inflammatory diseases.

### Brief Description of Drawings

Other features, details and advantages will be shown in the following detailed description and on the figures which comprise:
**Figure 1****:** In *vitro* expansion experimental protocol. Graft sources can be CBU (cord blood unit) or PBSC (peripheral blood stem cells). CD34 are purified from the Ficoll-purified peripheral blood mononuclear cell (PBMC) fraction by positive selection using CD34+ microbead kit and then cultured in medium specifically developed for the *in vitro* culture and expansion of human hematopoietic cells (for example the StemSpan) with cytokines (GM-CSF, G-CSF, SCF, TPO, FLT3-L) for 7 days.
**Figure 2****:** Percentage of e-MDSC in the CD34+ fraction after 7 days of culture. Median : 79,95% ; range : 59,7%-98,4% (n=19).

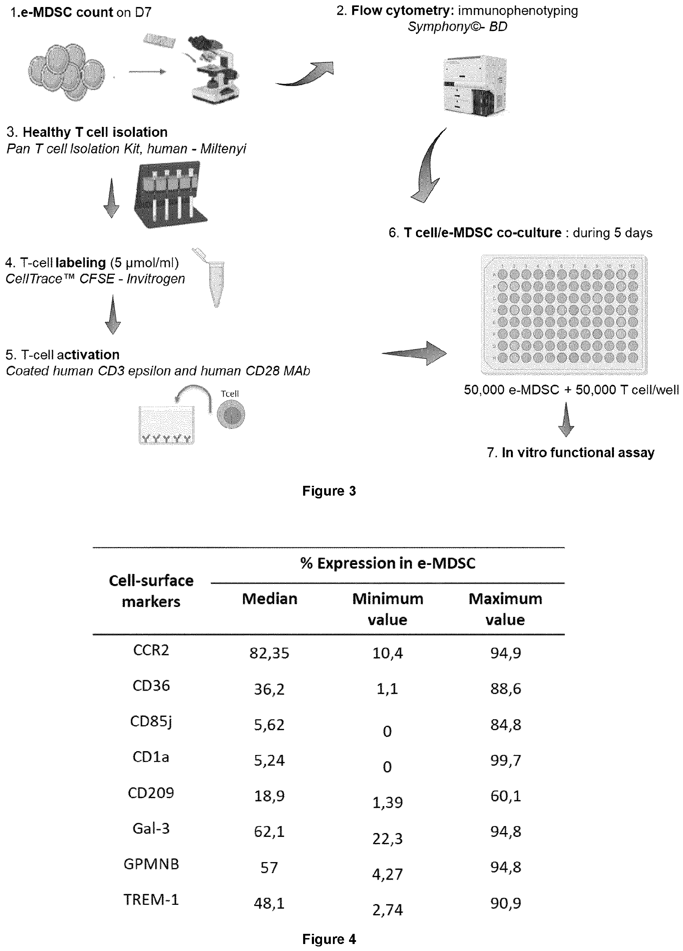

**Figure 3****:** Experimental protocol to validate the immunophenotype of e-MDSC after 7 days of culture (step 2) and to explore the immunosuppressive properties in vitro. T-cells are purified by negative selection using a pan T-cell depletion kit (Miltenyi Biotec, step3). Purity is routinely above 98%. T cells are stained with 5 µM carboxyfluorescein succinimidyl ester (CFSE) (Invitrogen, step 4). T-cell activation (50,000 cells/well) is performed by nonspecific TCR activation in a 96-well culture plate coated with 10 µg/mL anti-CD3 mAb (Biotechne) and 10 µg/mL anti-CD28 mAb (Biotechne) for 2 hours before the addition of T cells (step 5). e-MDSCs are plated at 50,000/well (1:1 ratio). Cells are incubated in custom RPMI 1640 with 10% fetal calf serum and 100 U/mL penicillin-streptomycin (Life Technologies). T-cell proliferation is analyzed after 5 days of coculture (step 6).
**Figure 4****:** Further exploration of cell-surface markers on e-MDSC by flow cytometry. CCR2, CD36, CD85j, CD1a, CD209, Gal-3, GPMNB, TREM-1 were explored based on a previous single cell analysis.
**Figure 5****:** Median survival in mice receiving PBMCs +eMDSC expanded *in vitro* from CBU or PBSC in comparison with median survival in mice receiving PBMC alone (Kaplan-Meier survival curves).

### Detailed Description of the Invention

The present invention thus pertains to a method for obtaining myeloid-derived suppressor cells (MDSCs), said method comprising a step of culturing CD34⁺ cells that have been isolated from a bone marrow graft or from peripheral blood or cord blood stem cells in a culture medium with cytokines, wherein said cytokines are TPO, G-CSF, GM-CSF, FLT-3L and SCF.

"Myeloid-derived suppressor cells" or "MDSCs" are cells of myeloid origin that consist of myeloid progenitor cells and immature myeloid cells. They have a suppressive power over various T-cell functions, and their activation in pathological conditions induces an increase in the expression of immune suppressive factors such as arginase and inducible nitric oxide synthase, and in the production of nitric oxide and reactive oxygen species. Accumulation of MDSCs has been observed in various diseases such as in cancer, autoimmunity and inflammation. In humans, their phenotype is Lin⁻HLA⁻DR⁻CD33⁺ or CD11b⁺CD14⁻CD33⁺ (see Gabrilovich D and Srinivas N, Nature reviews immunology 9.3 (2009): 162-174).

**"CD34"** or "**cluster of differentiation 34"** is a marker for pluripotent hematopoietic stem or progenitor cells. CD34 is a cell-surface glycoprotein type 1 transmembrane protein that belongs to the sialomucin family. CD34⁺ cells lose their hallmark CD34 expression upon differentiation into their destined lymphohematopoietic lineages.

According to the present invention, CD34⁺ cells can be isolated from a bone marrow graft, from peripheral blood stem cells (PBSCs) obtained from a donor, or from umbilical cord blood (UCB). Bone marrow grafts are generally obtained by punction using a needle placed into the marrow cavity of the hipbone, and peripheral blood stem cells (PBSCs) are generally collected by continuous-flow apheresis from the donor. CD34⁺ cells can be isolated from other cell populations via several techniques known by the skilled person. These techniques include e.g. using immunoaffinity (including fluorescence-activated cell sorting/FACS), immunomagnetic techniques and submicroscopic magnetic beads (see De Wynter et al., Bone marrow transplantation 23.11 (1999): 1191-1196). According to a preferred embodiment, CD34⁺ cells can be isolated by using magnetic cell isolation techniques. The skilled person is completely familiar with all these techniques and several kits and apparatus are currently available for performing them (such as e.g. the Dynabeads^{™} CD34⁺ Isolation Kit - Thermo Fisher; the CD34 MicroBead Kit - Miltenyi Biotec Inc; or the EasySep^{™} Human CD34 Positive Selection Kit - STEMCELL Technologies). The population of CD34⁺ cells can be obtained by aspiration of steady state bone marrow, by flushing blood from umbilical cord after delivery or by apheresis of peripheral blood after G-CSF mobilization. The administration of G-CSF is routinely performed for obtaining e.g. peripheral blood stem cells from healthy donors for allogenic stem cells transplantation (see e.g. Stroncek et al., Transfusion medicine 7.1 (1997): 19-24). Typically, G-CSF is administered subcutaneously at 10µg/kg/d for 3, 4 of 5 days.

According to a preferred embodiment, the "donor" in the context of the present invention is a healthy person, i.e. a healthy donor. The donor does thus not suffer from any illness. According to this embodiment, the MDSCs obtained according to the method of the present invention are used for allogenic transplantation.

The "**culture medium**" used for performing the method according to the method of the present invention is any physiologically acceptable medium that allows for the growth of MDSCs. Physiologically acceptable media are aqueous media that comprise electrolytes such as sodium potassium, magnesium and/or calcium salts, including anions such as chloride, carbonate, hydroxide or caprylate. A suitable culture medium that can be used for performing the method according to the present invention is e.g. the HSC Brew GMP Medium by Miltenyi or the StemSpan^{™} medium provided by StemCell Technologies or equivalent.

The method according to the present invention comprises culturing CD34⁺ cells with a specific combination of cytokines comprising TPO, G-CSF, GM-CSF, FLT-3L and SCF. The present inventors have indeed demonstrated that this specific cytokine mixture allows for the efficient and rapid production of myeloid-derived suppressor cells.

Thus, the present invention also pertains to the use of a combination of TPO, G-CSF, GM-CSF, FLT-3L and SCF for obtaining myeloid-derived suppressor cells. The present invention further pertains to a composition, in particular a culture medium as defined above, comprising CD34⁺ cells, TPO, G-CSF, GM-CSF, FLT-3L and SCF.

"**Thrombopoietin**" or "**TPO**" also known as megakaryocyte growth and development factor (MGDF) is a protein that in humans is encoded by the *THPO* gene. It is produced by the liver and kidney and regulates the differentiation of megakaryocytes and platelets. TPO can be easily obtained from specialized manufacturers and has an amino acid sequence accessible under reference P40225 in the Uniprot database.

"**Granulocyte-colony stimulating factor**" or "**G-CSF**", is a glycoprotein that regulates the production, maturation and release of neutrophiles in/from bone marrow. G-CSF can be easily obtained from specialized manufacturers and has an amino acid sequence accessible under reference P09919 in the Uniprot database.

"**Granulocyte-macrophage colony-stimulating factor**" or "**GM-CSF**" is a monomeric glycoprotein that stimulates the production of granulocytes and monocytes from stem-cells. GM-CSF is secreted by macrophages, T cells, mast cells, natural killer cells, endothelial cells and fibroblasts. It can be easily obtained from specialized manufacturers and has an amino acid sequence accessible under reference P04141 in the Uniprot database.

"**FMS-like tyrosine kinase 3 ligand**" or "**FLT-3L**" is a cytokine and growth factor that stimulates the proliferation of early hematopoietic cells, thereby increasing the number of T- and B-cells, by activating FLT3 (CD135). FLT-3L can be easily obtained from specialized manufacturers and has an amino acid sequence accessible under reference P49771 in the Uniprot database.

"**Stem cell Factor**" or "**SCF**", also referred to as "**KIT-ligand**" binds to the c-KIT receptor (CD117) and plays an essential role in the regulation of cell survival and proliferation, hematopoiesis, stem cell maintenance, gametogenesis, mast cell development, migration and function, and in melanogenesis. It can be easily obtained from specialized manufacturers and has an amino acid sequence accessible under reference P21583 in the Uniprot database.

The concentration of each cytokine in the culture medium is typically comprised between 75 and 125 ng/mL, preferably around 100ng/mL (i.e. comprised between 95 and 105 ng/mL). According to a specific embodiment, the concentration of each cytokine in the culture medium is 100ng/mL.

The culture of the CD34⁺ cells is typically performed under normoxic conditions i.e. in physiological oxygen concentrations. The cells are thus cultured in the presence of 21% of oxygen.

The culture of the CD34⁺ cells is typically performed at physiological temperature, i.e at a temperature comprised between 36 and 38°C, preferably 37°C.

The method according to the present invention allows obtaining myeloid-derived suppressor cells within 7 days.

The method according to the present invention can further advantageously comprise a step of screening the myeloid-derived suppressor cells obtained in order to specifically select the cells expressing CD34 at the end of the culturing step. This can be performed as described above, by using techniques such as immunoaffinity (including fluorescence-activated cell sorting/FACS) and immunomagnetic techniques. Selecting the CD34⁺ at the end of the culturing step allows obtaining e-MDSCs having the most efficient immune-suppressive properties.

According to a further embodiment, the present invention further pertains to the myeloid-derived suppressor cells obtainable from the method according to the present invention. The present inventors have shown that the myeloid-derived suppressor cells obtained according to the present invention are defined as being CD34+, CD11b+, CD33+, CD127+ and Lin- cells. Thus, according to a further embodiment, the present invention pertains to a myeloid-derived suppressor cell, wherein said cell expresses cell surface proteins CD34, CD11b, CD33 and CD127 while being Lin⁻ cells.

CD11b, CD33, CD127 and Lin are all well-known surface protein.

"**CD11b**", also known as "**ITGAM**" or "**Intergrin alpha M**", is an integrin molecule expressed on the surface of leukocytes which is implicated in various adhesive interactions of monocytes, macrophages and granulocytes as well as in mediating the uptake of complement-coated particles and pathogens. CD11b is one of the subunits forming the heterodimeric integrin alpha-M beta-2 (αMβ2) molecule, also known as macrophage-1 antigen (Mac-1) or complement receptor 3 (CR3). Its amino-acid sequence is accessible under reference P11215 in the UniProt database.

"**CD33**" or "**Siglec-3**" for "**sialic acid binding Ig-like lectin 3**" is a transmembrane receptor expressed on cells of myeloid lineage that plays a role in mediating cell-cell interactions and in maintaining immune cells in a resting state. Its amino-acid sequence is accessible under reference P20138 in the UniProt database.

"**CD127**" or "**interleukin-7 receptor-α**" is one of the two subunits of the interleukin-7 receptor. It is encoded by the IL7R gene, and its amino acid sequence is accessible under reference P16871 in the UniProt database.

"**Lin⁻**" refers to "**bone marrow-derived lineage-negative cells**", i.e. to cells which lack the expression of mature blood cell markers (especially CD3- and CD19-). The skilled person is familiar with this concept and knows how to identify cells falling under this definition (see e.g. Ramanauskaite, Giedre, et al. Turkish Journal of Biology 42.3 (2018): 205-212).

The skilled person knows several techniques that allow determining whether a cell expresses a specific cell-surface marker, including CD34, CD11b, CD33 and CD127. Typically, such techniques comprise a step of contacting the cells with at least one selective binding agent capable of selectively interacting with said marker. The selective binding agent may be a polyclonal antibody or a monoclonal antibody, an antibody fragment, synthetic antibodies, or other protein-specific agents such as nucleic acid or peptide aptamers. The binding agent may be directly coupled with detectable labels such as enzymes, chromogens or fluorescent probes or indirectly detected with a secondary binding agent conjugated with detectable labels.

The myeloid-derived suppressor cell accord to the invention can further express at least one cell surface protein selected from the group consisting of CCR2, CD36, CD85J, CD1a, CD209, GPMNB, GALECTIN-3, and TREM-1. All these markers are also well-known and readily identifiable by the skilled person.

The myeloid-derived suppressor cells obtained from the method as described above show a strong *in-vitro* and *in-vivo* immunosuppressive power and allow efficiently inhibiting T-cell responses. The cells produced in the context of the present invention therefore represent an extremely powerful tool for the treatment of diseases conditions involving a deleterious immune response, particularly a deleterious T-cell response. Such diseases/conditions include graft versus host disease or graft rejection, as well as autoimmune and inflammatory diseases.

Accordingly, a further aspect of the present invention pertains to the myeloid-derived suppressor cells obtained according to the present invention for use as a therapy, particularly for use in the treatment of graft versus host disease, graft rejection, or of an autoimmune or inflammatory disease in a patient.

"**Immune responses**" refer to the concerted action of lymphocytes, antigen presenting cells, phagocytic cells, granulocytes, and soluble macromolecules produced by the above cells or the liver (including antibodies, cytokines, and complement) that results in selective damage to, destruction of, or elimination from the body of a subject of target cells (such as cancerous cells, pathogens, cells or tissues infected with pathogens, or, in cases of autoimmunity or pathological inflammation, normal cells or tissues of a subject).

"**Graft versus host disease**" or "**GVHD**" is a complication following an allogenic transplant wherein the grafted tissue cells attack the host cells. There are two forms of GVHD: acute graft versus host disease and chronic graft versus host disease (see for review Blazar al., Nature reviews immunology 12.6 (2012): 443-458).

"**Graft rejection**" also referred to as "**host versus graft disease**" occurs when transplanted tissue/cells are rejected by the recipient's immune system, which results in the destruction of the transplanted tissue (see for review Van Dyke, Knox. "Transplantation Rejection.", 2014). Graft rejection can be hyperacute (i.e. occur within minutes-days after transplantation), acute (i.e. occur within 0 to 6 months after transplantation) or chronic (i.e. occur within 6 to 12 months after transplantation). It can affect any transplanted tissue/organ. In the context of the present invention, the graft is preferably a solid organ graft, more particularly kidney of heart graft.

Typically, when used for GVHD treatment or prophylaxis or in case of graft rejection, myeloid-derived suppressor cells obtained according to the present invention are administered to the patient who is the recipient of the graft. Cells are in suspension in a solution that could contain albumin 4%, NaCl 0,9%, ACD A formula. A single intravenous (IV) injection or repeated IV injections could be performed at a dose comprised between 0,1 to 1.10^6 MDSC/kg/injection.

An "**inflammatory disease**" is an expression that encompasses disorders and conditions that are characterized by inflammation. Examples include allergy, asthma, autoimmune diseases, coeliac disease, glomerulonephritis, hepatitis, inflammatory bowel disease, preperfusion injury and transplant rejection.

By "**autoimmune diseases**"**,** it is herein referred to conditions characterized by an abnormal immune response of the body against substances and tissues normally present in the body. In one particular embodiment, said autoimmune disease is selected from the group consisting of rheumatoid arthritis (RA), insulin dependent diabetes mellitus (Type 1 diabetes), multiple sclerosis (MS), Crohn's disease, systemic lupus erythematosus (SLE), scleroderma, Sjogren's syndrome, pemphigus vulgaris, pemphigoid, Addison's disease, ankylosing spondylitis, aplastic anemia, autoimmune hemolytic anemia, autoimmune hepatitis, coeliac disease, dermatomyositis, Goodpasture's syndrome, Graves' disease, Guillain-Barre syndrome, Hashimoto's disease, idiopathic leucopenia, idiopathic thrombocytopenic purpura, male infertility, mixed connective tissue disease, myasthenia gravis, pernicious anemia, phacogenic uveitis, primary biliary cirrhosis, primary myxoedema, Reiter's syndrome, stiff man syndrome, thyrotoxicosis, ulceritive colitis, and Wegener's granulomatosis.

As used herein, the terms "**treating**" or "**treatment**" refer to reversing, alleviating, inhibiting the progress of, or preventing the disorder or condition to which such term applies, or one or more symptoms of such disorder or condition. The terms "**prevention**" or "**preventing**" refer to alleviating the disease or condition from occurring in a subject which has not yet been diagnosed as having it.

The "**patient**" according to the present invention is a mammal, preferably a human.

Typically, when used for the treatment of an autoimmune or inflammatory disease, the myeloid-derived suppressor cells according to the present invention are administered to the patient in need thereof by intravenous injection. Route and number of infusions, time of administration, may be inspired from trials conducted with Mesenchymal Stromal Cells. e-MDSC doses can advantageously range from 0.1 to 1×10⁶ e-MDSC/kg, in 1 to 3 infusions, repeated every 3 to 7 days.

When used in the context of alloreactivity, e-MDSC doses can advantageously range from 0.1 to 1×10⁶ e-MDSC/kg, in 1 to 3 infusions, repeated every 3 to 7 days. Time of infusion are proposed at early stages of alloreactive diseases (both rejection or graft-versus-host disease). e-MDSCs are administered as prophylaxis for transplant rejection, or as a curative treatment early after first line treatment failure with an active inflammatory environment in order to prime e-MDSC for their immunosuppressive properties.

### EXAMPLES

Expansion protocol.

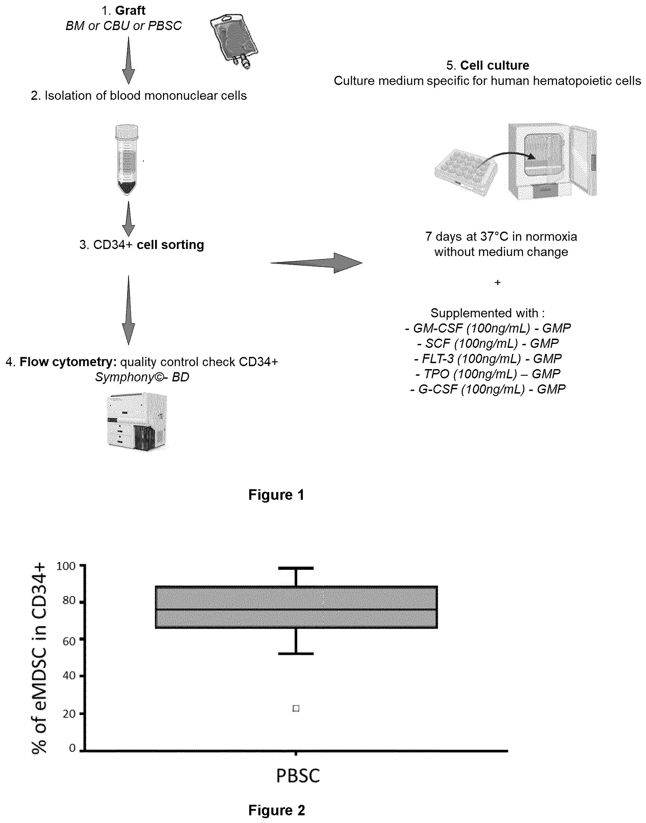

The protocol is as shown in Figure 1. Briefly, sorted CD34+ cells (Bead CD34⁺ miltenyi : CD34 MicroBead Kit human, ref: 130-046-703) from PBSCs are cultured at a concentration between 100 000 and 500 000 cells per mL in a GMP medium, for example the StemSpan (StemSpan^{™}-AOF cGMP medium, for culture and expansion of human hematopoietic cells, ref: 100-0130) and enriched with a GMP cytokine cocktail, at a final concentration of 100 ng/mL for each cytokine: GM-CSF : Human GM-CSF (50 µg) 130-095-372 , SCF : Human SCF (25 µg) 130-096-692, FLT-3 : Human Flt3-Ligand (25 µg) 130-096-474, G-CSF : filgrastim, TPO : romiplostim.

Cells are cultured for 7 days in normoxia (37°C, 5% CO2, 95% humidity). e-MDSC CD34⁺ cells are then counted, isolated and their immunosuppressive properties are tested in a coculture with activated T cells from healthy donor and e-MDSC CD34⁺. After 7 days, the percentage of eMDSC in CD34⁺ is shown in Figure 2.

The immunophenotype and immunosuppressive properties of the obtained e-MDSC are then evaluated as shown in Figure 3.

After exclusion of doublets the HLA-DR-/low Lineage (CD3-,CD19-) cells were gated. Then, in the gated HLA-DR-/low Lineage- a population of cells that expressed CD11b, and CD33 were identified. In this subset the CD34+ fraction (19.6%) were distinguished from the CD34- (80.6%). These cells are characterized by expression of CD127. At the end of the 7 days of culture, e-MDSC are gated as Lin-, CD11b+, CD33+, CD34+ and CD127+. A second CD34+ positive selection (which can be immunomagnetic or FACS sorting) allowed to sort 80% of e-MDSC (median on 19 sortings). Further exploration of cell-surface markers on e-MDSC by flow cytometry allowed determining that eMDSC further express CCR2, CD36, CD85j, CD1a, CD209, Gal-3, GPMNB at TREM-1 various levels (see Figure 4).

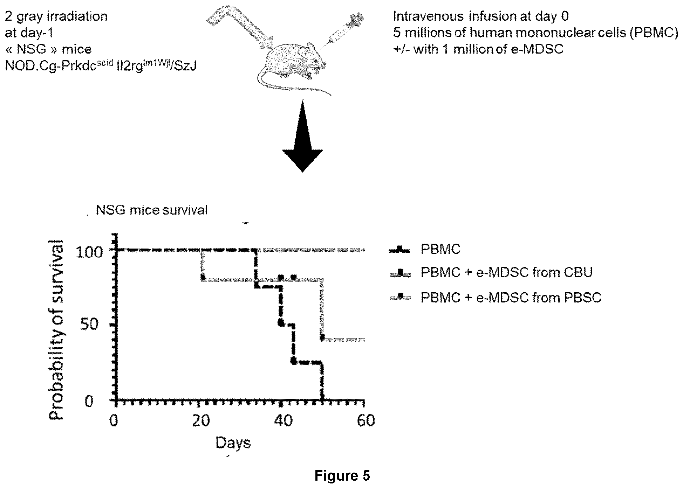

Administration of the eMDSC population according to the invention was then tested in murine models as shown in Figure 5. As shown in this figure, the median survival is of 41.5 days in mice injected with PBMCs alone (n=4). Median survival in mice receiving PMBC+ eMDSC CBU is not reached by day 60 (n=3). Median survival: 50 days in mice injected with PBMCs+ eMDSC PBSC (n=3). Mantel-Cox test. **P=0.0161.*

## Claims

1. A method for obtaining myeloid-derived suppressor cells, said method comprising a step of culturing CD34⁺ cells that have been isolated from a bone marrow graft or from peripheral blood or cord blood stem cells in a culture medium comprising cytokines, wherein said cytokines are TPO, G-CSF, GM-CSF, FLT-3L and SCF.

2. The method according to claim 1, wherein said bone marrow graft or peripheral blood stem cells are obtained from a healthy donor.

3. The method according to claim 1 or 2, wherein said culturing step is conducted under normoxic conditions at a temperature of 37°C.

4. The method according to any one of claims 1 to 3, wherein the concentration of each cytokine in the culture medium is comprised between 75 and 125ng/mL, preferably wherein the concentration of each cytokine is of 100ng/mL

5. A myeloid-derived suppressor cell, wherein said cell expresses cell surface proteins CD34, CD11b, CD33 and CD127 while being Lin⁻ cells.

6. The myeloid-derived suppressor cell according to claim 5, wherein said cell further expresses at least one cell surface protein selected from the group consisting of CCR2, CD36, CD85J, CD1a, CD209, GPMNB, GALECTIN-3, and TREM-1.

7. The myeloid-derived suppressor cell according to claim 5 or 6, wherein said cell is susceptible to be obtained by the method according to any one of claims 1 to 4.

8. The cell according to any one of claims 5 to 7, for use as a medicament.

9. The cell according to any one of claims 5 to 7, for use in the treatment of graft versus host disease or graft rejection.

10. The cell according to any one of claims 5 to 7, for use in the treatment of an autoimmune or inflammatory disease.

11. Use of a combination of TPO, G-CSF, GM-CSF, FLT-3 and SCF for obtaining myeloid-derived suppressor cells.

12. A composition comprising CD-34⁺ cells, TPO, G-CSF, GM-CSF, FLT-3 and SCF.
